# EUROPEAN PATENT APPLICATION

(11) **EP 1 199 668 A2**
(43) Date of publication of application: **24.04.2002**
(21) Application number: 00123925.0
(22) Date of filing: 03.11.2000
(51) Int. Cl.: G06F 19/00

(54) **A tool for investigating the properties of a substance**

(30) Priority: 20.10.2000 EP 00122872
(71) Applicant: LION Bioscience AG, 69120 Heidelberg (DE)
(72) Inventor: Etzold, Thure, Cambridge CB1 2DZ (GB); Vaughan, Brendan, Cambridgeshire PE18 8FA (GB)
(74) Representative: Betten & Resch

(57) **Abstract**

A computer-implemented tool for investigating the chemical and or biological properties of a substance comprising a sequence of nucleic acids or amino acids, said tool comprising means for displaying in a graphical display scheme at least one substance having at least one sequence similar or identical to a sequence of the substance to be investigated in such a manner that similar and/or identical sequences are respectively displayed through a corresponding first graphical indication the position of which with respect to a first dimension corresponds to the location of said similar sequence in said substance, and means for displaying a second graphical indication corresponding to each first graphical indication and indicating a domain information corresponding to the sequence indicated by said first graphical indication.

## Description

### FIELD OF THE INVENTION

The present invention relates to a tool for investigating the chemical or biological properties of substances.

### DESCRIPTION OF THE RELATED ART

Scientists in the field of biology or biochemistry often encounter new substances which they have isolated or otherwise identified and for which they want to know more about their behavior, their capabilities, their functions, etceteras.

For that purpose it is common to consult databases in which a large volume of information with respect to substances, *i.e.* for example their structure but also at least some of their properties and functions. It is general knowledge that similar structural parts may be responsible for a similar behavior, similar properties and similar functions, and this is the basic reasoning behind the consulting of such databases.

There exist many databases in which nucleic acid sequences or amino acid sequences are stored, often together with an identifier and together with some additional information further characterizing the stored sequence with respect to ist structure or its function. As an example, a nucleic acid may contain repetitive elements such as multiple copies of the three nucleotides "A, C, and A", e.g. "ACA ACA ACA". The annotation of the sequence with respect to the content of such a repetitive element could most likely be stored as an additional information in a database in which the corresponding sequence is stored.

Other elements may involve a particular function, such as the capability of a protein to metabolize a substrate.

Many proteins, also called enzymes if they exhibit a function, show numerous features or elements of which much may be known. In proteins, such functional elements are referred to as "domains". E.g., proteins that are capable of binding a DNA molecule will contain a "DNA binding domain".

Throughout the various organisms one may find enzymes that perform similar functions. Often such a function may be deduced by comparing protein domains.

One possibility could be to directly search for known domains in the substance to be investigated. This may lead to an initial understanding about the properties of the substance to be investigated, since identified domains give an indication about a certain function.

An alternative method follows a more elementary approach, namely to search for such substances which have sequences bearing similarities with sequences of the substance to be investigated. An unknown substance, such as a protein, a gene, an enzyme, or the like is used as a query string inputted into a database, in other words, the sequence of the unknown substance is used as input. It is then searched for substances the sequences of which bear some similarities with the query sequence, hoping that this will give the scientists a hint about the properties of the unknown substance.

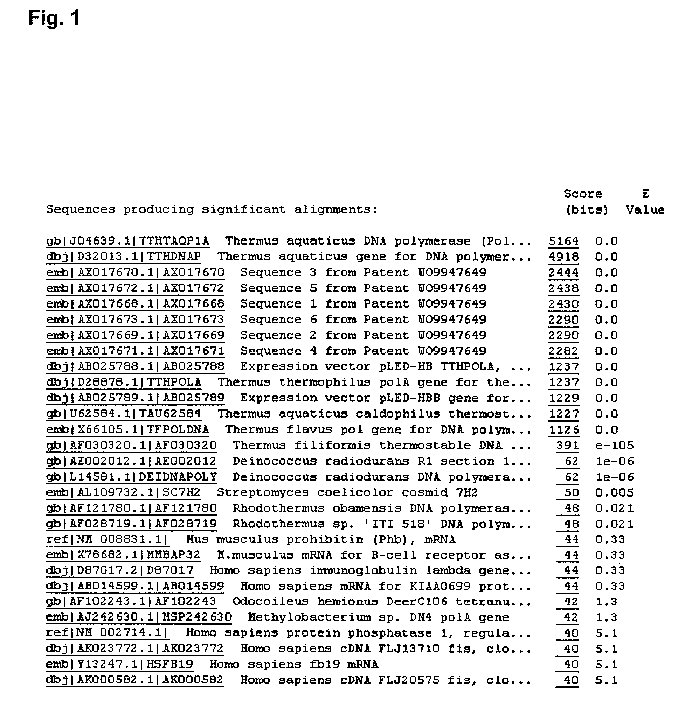

The output of such a query usually consists of a list of sequences together with a corresponding name or identifier as well as a numerical figure indicating the degree of coincidence between query and result. This is shown in Fig. 1.

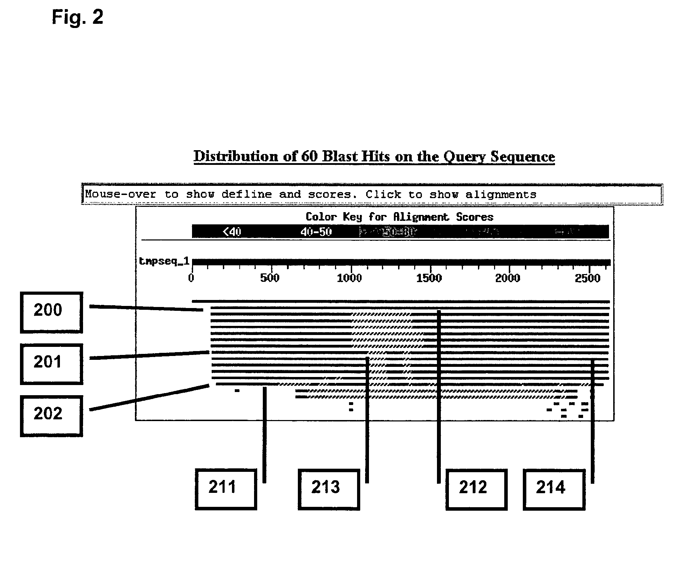

In some cases there is also a graphical indication in the result display as to where the similar sequences are located in order to thereby give some graphical indications as to where the two sequences share character states (domains). This is schematically illustrated in Fig. 2, where the result sequences 200, 201, 202 comprise indications 211, 212, 213, 214 as to where the similarities with the query sequence are located.

Given the previous approaches, there is a need for an improved tool for investigating the properties of unknown substances. A substantial problem is that the chemical and biological properties of a substance comprising a sequence of nucleic acids or amino acids are not readily apparent when a database output is analyzed, in particular with respect to their distribution within a substance of interest but also within related substances of interest.

### SUMMARY OF THE INVENTION

The present invention provides a tool for investigating the properties of a substance based on similarities of said substance with known substances. This can be achieved by not only displaying first graphical indications of locations of similar sequences but also second graphical indications of domains corresponding to said similar sequences. Herein, domains are to be understood as regions of distinct structure to which biological or chemical information is available. In the case of peptides such domains may be independent regions of the polypeptide chain, which can on the one hand be distinguished by the way they are folded (structure of space) and their movability, and which, on the other hand, often have separate functions within the protein and represent genetic modules which are combined to different genes and thus proteins. In the case of nucleic acids such domains are elements encoding the above peptide domains or other distinct elements found in nucleic acids, such as repetitive elements or the like. Thus, in its broadest definition a domain is a distinct feature element for which some information is available, e.g. about its function or distribution amongst the various organisms on earth. Thereby, it becomes possible for the scientists to grasp at a single glance an hint with respect to the behavior of an unknown substance. Rather than giving only statistical information as in the prior art, the scientist is provided with actual "biological" or "biochemical" information about the probable behavior of the substance to be investigated. Generally, if a novel protein or a novel gene coding for a protein is found and the function of which is so far unknown, it is a good approach to start by looking at which domains may be identified. Based on this knowledge, the function of the protein may be rendered obvious or at least there may be provided significant hints for an initial understanding of the protein's function.

Preferably, the first graphical indication has the form of a line, the location of the line indicating the location of the similar sequence, an the length of the line indicating the length of the similar sequence. This makes it easy to get at a first glance an impression about which of the similar sequences may be give significant hints to corresponding properties (the longer ones) and where they are located.

Preferably, the first graphical indications are for different sequences located at different positions in a second dimension, such as the y-axis. This makes the display particularly easy to look at, with none of the first graphical indications overlapping each other.

Preferably, the second graphical indication has the form of a pop-up window which when clicking on a first graphical indication pops up and delivers further information regarding the domain corresponding to said similar sequence.

The sequence of the substance to be investigated is preferably used as an input to query a database which then returns substances having similar sequences. The results may then be displayed as indicated above.

The present invention will now be explained in more detail trough exemplary embodiments in connection with the accompanying drawings.

### BRIEF DESCRIPTION OF THE DRAWINGS

- Fig. 1: shows a display scheme according to the prior art.A query sequence has been sent to a database as a query string input namely to search for such substances which have sequence parts bearing similarities with the sequence to be investigated. The display according to the prior art is the result returned.
- Fig. 2: shows another display scheme according to the prior art.ln this case the regions are depicted which show similarity.
- Fig.3: shows a database query used in connection with an embodiment of the invention. In this preferred embodiment of the invention, the query string is a sequence. It is used to initiate a search for similar sequences in the database. In another embodiment, a unique identifier is sent off as a query string.
- Fig. 4: shows a display scheme according to an embodiment of the present invention.
- Fig. 5: shows a display scheme for a further result according to an embodiment of the present invention

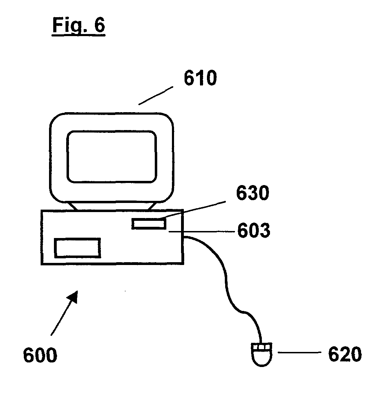

- Fig. 6: schematically illustrates a computer system to be used in connection with an embodiment of the present invention.

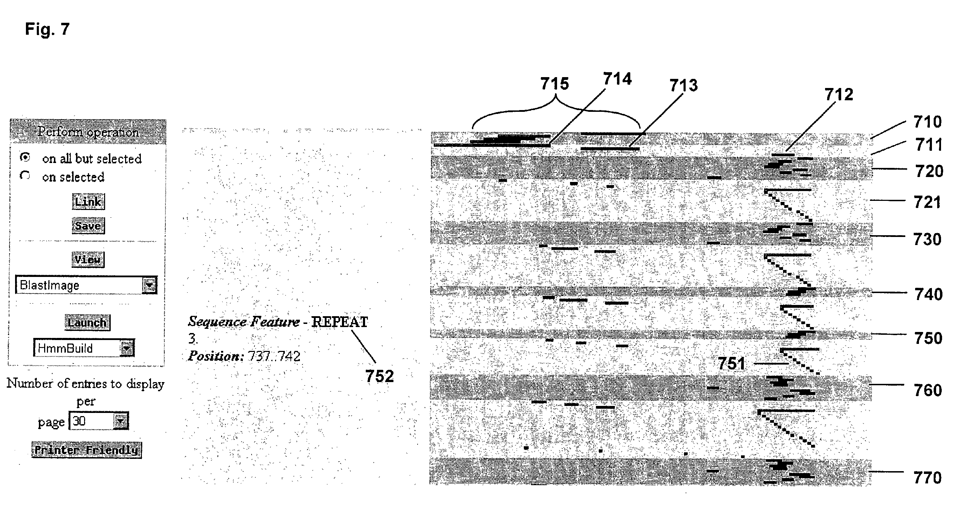

- Fig. 7: shows an output of the computer-implemented tool according to the invention. Here, the query has resulted in the return of sequences 710, 720, 730, 740, 750, 760, and 770. In the case of "result" sequence 710, 710, the darker bar designates and comprises regions of similarity 715.
A mouse over would result in a pop-up or alternatively a description to the right showing the highest score pairs (HSP) regions. Bars 712, 713 and 714 represent features or domains contained within the sequence. A mouse-over results in a visual description of the location and characteristics of the domains. For sequence 750 a mouse-over has been performed at position 751, resulting in description 752. It is equally possible to double click on any of the feature bars, *e.g*. 714, 713, 712 and 751 and, thereby arrive at a page providing further information.

### DETAILED DESCRIPTION

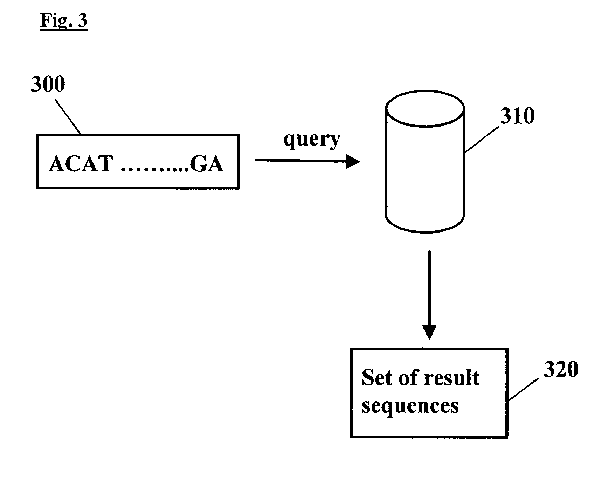

Fig. 3 schematically shows a situation in which an embodiment of the present invention may be used. A substance 300 has to be investigated with respect to its biological or biochemical properties. It consists of a nucleic acid sequence comprising nucleotides 300 or alternatively an amino acid sequence comprising amino acids.

In order to investigate the substance it is used as a an input to query a database 310 in which a large volume of sequences is stored, possibly with additional information for each sequence which may contain not only its name or identifier, but also additional information with respect to the domains known for the sequences stored, their corresponding functions, etceteras.

The query procedure can be carried out in a manner which is easy to implement by any person skilled in the art, it may use any database technology conventionally known and is therefore not explained in detail here. It should only be mentioned that the particular search strategy or search conditions, such as for which degree of similarity between query sequence and a dataset stored in database 310, "a hit", i.e. a query result is returned, depends on the methodology used for querying, *i.e*. in a preferred embodiment this is a similarity search tool that searches the database. The query result returned also depends on the content of the database. In a preferred embodiment this similarity search tool is the BLAST algorithm.( Altschul, Stephen F., Warren Gish, Webb Miller, Eugene W. Myers, and David J. Lipman (1990). Basic local alignment search tool. J. Mol. Biol. 215:403-10.

Another search tool that may be used is FASTA (W. R. Pearson and D. J. Lipman (1988), "Improved Tools for Biological Sequence Analysis", PNAS 85:2444- 2448, and W. R. Pearson (1990) "Rapid and Sensitive Sequence Comparison with FASTP and FASTA" Methods in Enzymology 183:63- 98).

The invention is not limited to one particular type of search tool. In one embodiment of the invention search tools are used that do not search by sequence similarity but by applying sequence profiles such as a profile generated when applying the Profile Hidden Markov Model.

Profile Hidden Markov Models also called "Hidden Markov Models", here abbreviated as HMM, are statistical models representing the consensus of the primary structure of a sequence family. The profiles use scores specific of the position of amino acids (or nucleotides) and position specific scores for the opening or the expansion of an insertion or deletion. Methods for the creation of profiles, starting from multiple alignments, have been introduced by Taylor (1986), Gribskov et al. (1987), Barton (1990) and Heinikoff (1996).

HMMs provide an utterly probabilistic description of profiles, *i.e*. Bayes' theory rules the positioning of all probability (evaluation) parameters (compare Krogh et al. 1994, Eddy 1996 und Eddy 1998). The central idea behind this is that a HMM is a finite model describing the probability distribution of an infinite number of possible sequences. The HMM consists of a number of states corresponding with the columns of a multiple alignment as it is usually depicted. Each state emits symbols (remainders) corresponding with the probability of the symbol emission (specific of the respective state), and the states are linked with each other by probabilities of the changing of states. Starting from one specific state, a succession of states is generated by changing from one state to the other in accordance with the probability of the changing of states, until a final state has been reached. Each state then emits symbols according to the probability distribution of emissions specific of this state, creating an observable sequence of symbols.

The attribute "hidden" has been derived from the fact that the underlying sequence of states cannot be observed. Only the sequence of symbols is visible. An assessment of the probabilities of changing of states and of emissions (the training of the model) is achieved by dynamic programming algorithms implemented in the HMMER package.

If an existing HMM and a sequence are given, the probability that the HMM could generate the sequence in question, can be calculated. The HMMER package provides a numerical quantity (the Score) in proportion to this probability, *i.e.* the information content of the sequence indicated as bits, measured according to the HMM.

See also Barton, G.J. (1990): Protein multiple alignment and flexible pattern matching, Methods Ezymol. 183: 403-427, Eddy, S.R. (1996): Hidden markov models. Curr. Opin. Strct. Biol. 6: 361-365, Eddy, S.R. (1998): Profile hidden markov models.Bioinformatics. 14: 755-763, Gribskov, M. McLachlan, A.D. und Eisenberg D. (1987): Profile analysis: Detection of distantly related proteins. Proc. Natl. Acad. Sci. USA. 84: 4355-5358, Heinikoff, S. (1996): Scores for sequence searches and alignment, Curr. Opin. Strct. Biol. 6: 353-360, Krogh, A., Brown, M., Mian, I.S., Sjolander, K. und Haussler, D. (1994): Hidden markov models in computational biology: Applications to protein modelling. J. Mol. Biol. 235: 1501-1531, Taylor, W.R. (1986): Identification of protein sequence homology by consensus template alignment. J. Mol. Biol. 188: 233-258.

In general the sequence are selected such that a query using a search sequence returns a result consisting of sequences which are at least to a certain degree similar to the query sequence.

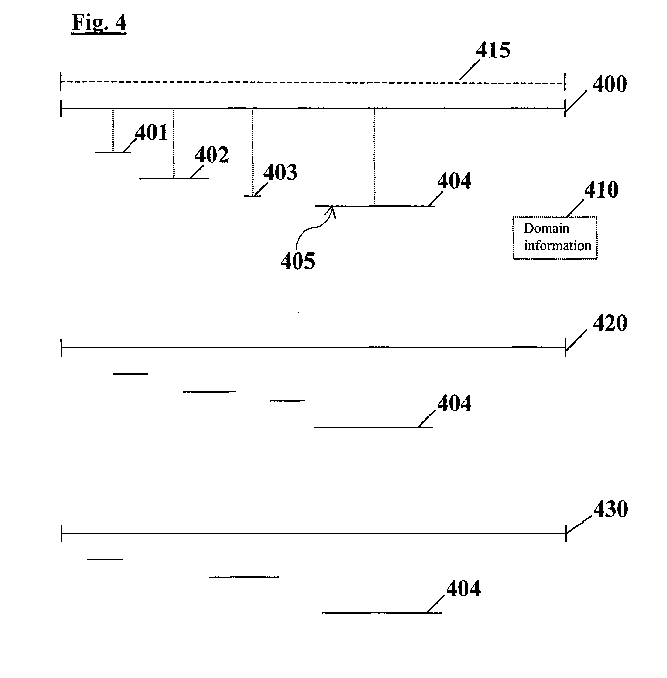

After one or more result datasets have been returned by database 310, they are displayed as schematically illustrated in Fig. 4.

The line 400 shown in Fig. 4 indicates a first hit returned from a database query based on a query string. Two other query results 420 and 430 are also shown in Fig. 4. Result 400 consists of a sequence which has been found as bearing at least some similarity to the sequence used as query sequence. The query string itself may be displayed in a separate window not shown in Fig. 4.

For result 400 there are four (partial) sequences in which result 400 is similar to the query string, they are graphically indicated by the lines 401, 402, 403, and 404. In order to avoid an overlap between lines 401 to 404 they are respectively located at different positions in the x-direction. The length of those lines indicates the extent of coincidence with the query string, in case of sequence 404 there is e.g. a large degree of coincidence between query and result, to the contrary, in case of sequence 403 the degree of coincidence is relatively small. Degree of coincidence here means the length of a sequence part in which query and result coincide with each other.

There is still further information which a scientist can derive from the display scheme shown in Fig. 4, namely where in the query the similar sequences are located. This already may give some indication about the function of some of the sequences, the information can be derived from the respective centers of lines 401 to 404, as indicated by the dashed lines in Fig. 4. The query sequence itself may also be displayed such as by the dashed line 415 in Fig. 4, although this is actually not necessary. The results 400, 420 and 430 are preferably normalized in their length in the y-direction to the length of the query sequence.

Additional information with respect to the domains (domain information) related to the similar sequences is displayed for example in a pop-up window 410 which shows up when e.g. mouse pointer 405 moves close to line 404 (when the mouse comes over line 404) or when the user clicks on line 404. Then the user is provided with additional information regarding the domain(s) related to the sequence identified as similar. Such information may contain the function name of that domain, it may also contain additional information about ist structure, or any information stored in a database about that domain.

From that information a scientist can derive extremely valuable hints as to which are the functions or properties of the unknown substance. If e.g. the results show that the query and the results share the "DNA binding domain" as the domain information, then this gives an indication that the unknown substance is a protein that binds DNA rather than, *e.g*. digesting (cutting) it. In the popup window not only domain information may be provided. It may happen that a sequence part found to be similar does not belong to a certain domain but rather has other features or character states such as that it contains a repetitive element or the like. This kind of information could show up as well in the popup window. The popup window may contain any information found in the database about the element or domain which has been found to be similar to the query sequence. It should be noted that a popup window is only the preferred graphical representation of the additional information provided. Other possibilities are, e.g. depicting the information directly in the same window.

In Fig. 4 three hits (results) 410, 420, 430 together with additional graphical information regarding the location of similar sequences and the corresponding domains are schematically illustrated. In a preferred embodiment, particular sequence features, e.g. domains, may be attributed defined graphical representations.

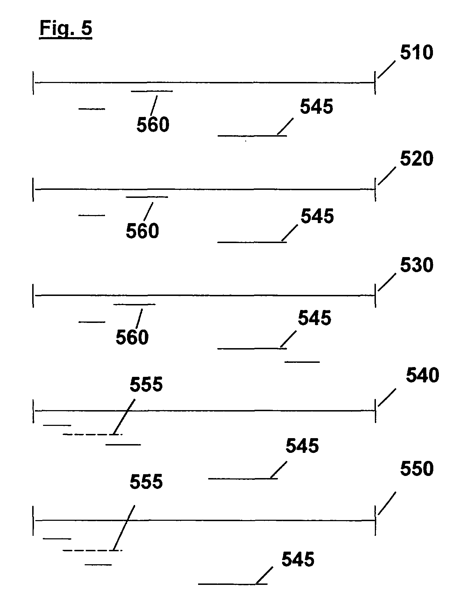

Fig. 5 illustrates another example of a result provided by the computer implemented tool according to the invention. It becomes readily apparent that the result set 520, 530, 540, and 550 obtained for the query sequence 510 contains two groups of results. In the first group, results 520 to 550 all contain a sequence 545 corresponding to a DNA binding domain, as becomes apparent from a popup window (not shown), which shows up when the cursor moves to one of the sequences 545. The other group, 540 and 550, however, contain a domain 555 corresponding to a DNA polymerization domain, *i.e*. activity for synthesizing DNA.

Moving the mouse over domain 560 will result in the information that this is a domain found in enzymes that digest (degrade) DNA.

By applying the invention, the user has thus learned that there are two groups of substances that are similar to the query substance, having in common the capability to bind DNA. A first group capable of digesting DNA, to which the query sequence belongs, and a second group capable of synthesizing DNA.

In another embodiment (not shown here) by using a user option a user may change the display of Fig. 5 such that instead on only similar sequences or domains all the domains known for results 510 to 550 are displayed. This would then result for each of the sequences 510 to 550 in a display as if it would have been itself the query sequence. With this option a user may then e.g. find that sequences 540 and 550 contain domains 570 which are responsible for a different function such as an "intein" (an interspersed element) or any other known element. The similarity between those results and the query sequence, however, clearly is not based on a similarity regarding this function but on other similarities.

Another user option may be that a user can choose to get displayed all the sequences in the query which belong to already identified domains, irrespective of the result sequence in which they occur. Basically this would consist in replacing lines 510 to 550 by a line representing a query and further by displaying all domains displayed in Fig. 5 more than once just once. This would give a concise display of all domains related to the query, although this scheme of displaying may become quite complicated to overlook if many domains (or corresponding sequences) are to be displayed.

Many variations to the above-mentioned embodiment can be imagined. For example not only the domains for which a similarity of sequences between query and result exits could be displayed, but in general all domains known for a found result. This could e.g. be provided as a further option only to be displayed when requested by the user.

It can e.g. also be imagined that different colors are used for lines 401 to 404 indicating similar sequences, the colors e.g. coding for the most frequent domains or functions, such as DNA binding or not DNA binding, or the like. Then even more information could be directly derived from a display as shown in Fig. 4, even without a pop-up window. Which color codes for what may thereby be different from implementation to implementation.

There may also be provided a further window in which e.g. the name of the query and the sequences found may be listed, possibly such that the sequence where the mouse is located in Fig. 4 is the one also shown as the first one in the additional window.

Embodiments of the present invention may be implemented using a computer system as schematically illustrated in Fig. 5. A computer system 600 may comprise a computer 605 connected to a display 610, a mouse 620, and comprising some storage medium 630 such as a floppy disk drive, a CD-ROM drive or the like, and some hardware components 640 comprising at least one CPU and a memory such as to enable the computer to carry out by means of the CPU program instructions stored in said memory. The program itself may be stored on any computer readable medium, or it may by carried out on a remote computer (host) to be accessed by a client computer through a communications link such as the internet. Hybrid implementations are also possible, such as a Java implementation being downloaded in part or as a whole through a network and carried out on a client.

## Claims

**1.** A computer-implemented tool for investigating the chemical and or biological properties of a substance comprising a sequence of nucleic acids or amino acids, said tool comprising:
means for displaying in a graphical display scheme at least one substance having at least one sequence similar or identical to a sequence of the substance to be investigated in such a manner that similar and/or identical sequences are respectively displayed through a corresponding first graphical indication the position of which with respect to a first dimension corresponds to the location of said similar sequence in said substance; and
means for displaying a second graphical indication corresponding to each first graphical indication and indicating a domain information corresponding to the sequence indicated by said first graphical indication.

**2.** The computer-implemented tool according to claim 1, wherein
said first graphical indication has the form of a line, the length of said line indicating the length of said similar sequence, and the position of said line indicating the location of said similar sequence.

**3.** The computer-implemented tool according to claim 1 or 2, wherein
said first graphical indications for different sequences are located at different positions in a second dimension.

**4.** The computer-implemented tool according to one of the preceding claims, wherein
said second graphical indication is a pop-up window showing up in response to an action performed by a user.

**5.** The computer-implemented tool according to one of the preceding claims, wherein
said one or more substances having sequences similar or identical to the substance to be investigated result from a query of a database using said substance to be investigated as the query input for finding through said query said one or more substances having similar or identical sequences.

**6.** The computer-implemented tool according to claim 5, wherein
said query is performed using a method chosen from the group comprising, BLAST, FASTA and/or the Profile Hidden Markov Model.

**7.** The computer-implemented tool according to one of the preceding claims, further comprising:
means for displaying in response to a user request for a result sequence
all domains contained in the result set, such as if the result would itself have been the query sequence, and/or
means for displaying in response to a user request all domains identifiable in said query sequence.

**8.** A method for investigating the chemical and or biological properties of a substance comprising a sequence of nucleic acids or amino acids, said method comprising:
means for displaying in a graphical display scheme at least one substance having at least one sequence similar or identical to a sequence of the substance to be investigated in such a manner that similar and/or identical sequences are respectively displayed through a corresponding first graphical indication the position of which with respect to a first dimension corresponds to the location of said similar sequence in said substance; and
means for displaying a second graphical indication corresponding to each first graphical indication and indicating a domain information corresponding to the sequence indicated by said first graphical indication.

**9.** The method according to claim 8, wherein
said first graphical indication has the form of a line, the length of said line indicating the length of said similar sequence, and the position of said line indicating the location of said similar sequence.

**10.** The method according to claim 8 or 9, wherein
said first graphical indications for different sequences are located at different positions in a second dimension.

**11.** The method according to one of claims 8 to 10, wherein
said second graphical indication is a pop-up window showing up in response to an action performed by a user.

**12.** The method according to one of claims 8 to 11, further comprising
querying a database with said substance to be investigated as an input to obtain as a result one or more substances having sequences similar or identical to sequences of said substance to be investigated.

**12.** The method according to claim 12, wherein
said query is performed using a method chosen from the group comprising, BLAST, FASTA and/or the Profile Hidden Markov Model.

**13.** The method according to one of claims 7 to further comprising
displaying in response to a user request for a result sequence
all domains contained in the result set, such as if the result would itself have been the query sequence, and/or
displaying in response to a user request all domains identifiable in said query sequence.

**14.** A computer program comprising computer program code for enabling a computer to carry out a method according to one of claims 6 to 12.
